# EUROPÄISCHE PATENTANMELDUNG

(11) **EP 4 810 478 A1**
(43) Veröffentlichungstag der Anmeldung: **23.09.2026**
(21) Anmeldenummer: 25165275.6
(22) Anmeldetag: 21.03.2025
(51) Int. Cl.: C07C 253/10, C07C 255/46

(54) **VERFAHREN ZUR HERSTELLUNG VON ISOPHORONITRIL**

(71) Anmelder: Evonik Operations GmbH, 45128 Essen (DE)
(72) Erfinder: METTERNICH, Jan Benedikt, 45657 Recklinghausen (DE); NITZ, Jörg-Joachim, 45257 Essen (DE); MENDORF, Matthias, 44225 Dortmund (DE); MÜLLER, Thomas, 63674 Altenstadt (DE); GLÖCKLER, Bernd, 63486 Bruchköbel (DE); JOHN, Christian, 44625 Herne (DE)
(74) Vertreter: Evonik Patent Association

(57) **Zusammenfassung**

Gegenstand der vorliegenden Erfindung ist ein Verfahren zur Herstellung von Isophoronnitril, bei dem ein Gemisch umfassend die Wertstoffe a) Isophoron und b) mindestens einen keto- und ggf. hydroxylgruppenhaltigen Kohlenwasserstoff mit einem Molekulargewicht von 178 - 276 g/mol mit Cyanwasserstoff zu Isophoronnitril umgesetzt wird.

## Beschreibung

Der Gegenstand der vorliegenden Erfindung ist ein Verfahren zur Herstellung von Isophoronitril.

Isophoronnitril ist eine bedeutende Industriechemikalie, deren Relevanz unter anderem darauf beruht, dass sie als Edukt für die Synthese von als Epoxyhärter eingesetztem Isophorondiamin und des daraus herstellbaren Isophorondiisocyanats verwendet wird, das von großer Relevanz für die Polyurethanchemie ist. Aus diesem Grund sind Verfahren zur Herstellung von Isophoronnitril von großem Interesse. Üblicherweise wird Isophoronnitril aus Isophoron hergestellt.

Isophoron lässt sich basenkatalysiert aus Aceton synthetisieren: So offenbart zum Beispiel GB 733,650 A, dass Aceton basenkatalysiert zu Isophoron und höheren Kondensationsprodukten umgesetzt werden kann. In einer bevorzugten Ausführungsform werden die höheren Kondensationsprodukte in das Reaktionsgemisch zurückgeführt.

DE 1 205 525 A1 offenbart, dass bei der Kondensation von Aceton zu Isophoron entstandene Nebenprodukte mit wässriger Alkalilösung bei höheren Temperaturen behandelt und so niedrig siedende Stoffe, insbesondere Aceton, zurückgewonnen werden können. Das beschriebene Verfahren wird auch als Hydrolyse bezeichnet.

DE 26 45 281 A1 offenbart ein Verfahren zur Herstellung von Isophoron, bei dem eine Mischung aus Aceton, Wasser und einem Alkalikatalysator in einer Kolonne umgesetzt wird und vom Boden der Reaktionszone eine Isophoron, Wasser und nicht umgewandeltes Aceton enthaltende Fraktion zu einer Hydrolysezone geführt wird, von wo aus Aceton in die Reaktion rückgeführt und Isophoron abgeführt wird.

DE 10 2010 062 587 A1 befasst sich mit der Aufgabe, auf die Entstehung unerwünschter Nebenprodukte wie Diacetonalkohol, Mesityloxid, Phoron, Mesitylen und Überkondensate zurückzuführende verminderte Ausbeuten zu verbessern und lehrt, einen Isophoron und höhere Kondensate enthaltenden Wertstrom einer Hydrolyse zu unterwerfen, um die höherkondensierten Produkte teilweise oder vollständig in Isophoron, Aceton und andere Wertprodukte zu überführen. Der aus der Hydrolyse ausgeführte Wertstrom wird einer Phasentrennung unterworfen. Aus der dabei erhaltenen organischen Phase wird Isophoron isoliert und mit üblichen Methoden aufgereinigt.

CN 103435461 B offenbart ein Verfahren zur Herstellung von Isophoron aus Aceton, bei dem Aceton in Gegenwart eines alkalischen Katalysators zu einem Produktgemisch umfassend Wasser, Mesityloxid, Isopropyliden-Isomeren, Mesitylen, Isophoron und Tetramerisationsprodukten umgesetzt wird. Nach Abtrennung und Rückführung von Aceton sowie Abtrennung der leichteren Komponenten werden die Schwersieder (umfassend Isophoron und Tetramerisationsprodukte) aufgereinigt und Isophoron in einer Reinheit von 99 % erhalten.

CN 102367223 B offenbart ein Verfahren zur Herstellung von Isophoron, bei dem Aceton und Wasser in Gegenwart eines Katalysators umgesetzt, das Reaktionsgemisch hydrolysiert und einer Phasentrennung unterzogen wird. Bei der Hydrolyse werden Hochsieder mit 12 oder mehr Kohlenstoffatomen sowie nicht reagiertes Aceton vom Isophoron abgetrennt. Aceton wird anschließend in die Reaktion zurückgeführt.

Weiterhin sind Verfahren zur Herstellung von Isophoronnitril im Stand der Technik beschrieben:
DE 39 42 371 C2 offenbart ein Verfahren zur Herstellung von Isophoronnitril, bei dem Cyanwasserstoff in Gegenwart einer Alkaliverbindung als Katalysator an Isophoron angelagert wird.

U 5,011,968 A offenbart ein Verfahren zur Herstellung von Isophoronnitril, bei dem Isophoron in Gegenwart eines quaternären Ammoniumhydroxid-Katalysators mit Cyanwasserstoff umgesetzt wird.

Auch WO 2004/056753 A1 offenbart ein Verfahren zur Herstellung von Isophoronnitril, bei dem Isophoron in Gegenwart einer Base als Katalysator mit Cyanwasserstoff umgesetzt wird.

WO 2012/076317 A1 offenbart im Kontext einer Synthese von Isophorondiamin ein Verfahren zur Herstellung von Isophoronnitril, bei dem zunächst durch katalysierte Aldol-Kondensation von Aceton Isophoron hergestellt wird, das Reaktionsprodukt aufgearbeitet, der Wertstrom hydrolysiert und in eine organische und eine wässrige Fraktion getrennt wird und das Isophoron mit üblichen Aufreinigungsverfahren, bevorzugt durch Destillation, aus der organischen Fraktion gewonnen wird.

Nachteilig insbesondere an dem in WO 2012/076317 A1 offenbarten Verfahren ist, dass die zur Aufreinigung von Isophoron eingesetzten Verfahren, insbesondere die als bevorzugt genannte Destillation, sehr energieintensiv ist und somit ganzheitlich betrachtete auch bei der Synthese von Isophoronnitril zu einer ungünstigen Energiebilanz führen. Weiterhin ist nachteilig, dass die im Stand der Technik bekannten Verfahren zur Herstellung von Isophoronnitril nicht genügend hohe Ausbeuten und Selektivitäten aufweisen. Aufgabe der vorliegenden Erfindung ist es somit, die Energiebilanz sowie die Ausbeuten und Selektivitäten bei der Synthese von Isophoronnitril zu verbessern.

Diese sich vorliegend stellende Aufgabe wird gelöst durch das erfindungsgemäße Verfahren zur Herstellung von Isophoronnitril, bei dem ein Gemisch umfassend die Wertstoffe
(1) Isophoron und
(2) mindestens einen keto- und ggf. hydroxylgruppenhaltigen Kohlenwasserstoff mit einem Molekulargewicht von 178 - 276 g/mol
mit Cyanwasserstoff zu Isophoronnitril umgesetzt wird.

Gegenstand der vorliegenden Erfindung ist ein Verfahren zur Herstellung von Isophoronnitril, bei dem ein Gemisch umfassend die Wertstoffe Isophoron und mindestens einen keto- und ggf. hydroxylgruppenhaltigen Kohlenwasserstoff mit einem Molekulargewicht von 178 - 276 g/mol mit Cyanwasserstoff zu Isophoronnitril umgesetzt wird. Somit kann ein Teil des erzeugten Isophoronnitrils aus der Umsetzung von Isophoron mit Cyanwasserstoff (HCN) stammen. Zumindest partiell kann das erzeugte Isophoronnitril auch aus der Umsetzung des mindestens einen keto- und ggf. hydroxylgruppenhaltigen Kohlenwasserstoffs mit einem Molekulargewicht von 178 - 276 g/mol mit Cyanwasserstoff (HCN) stammen, wobei überraschenderweise festgestellt wurde, dass der mindestens eine keto- und ggf. hydroxygruppenhaltige Kohlenwasserstoff mit einem höheren Molekulargewicht als Isophoron vorhandene Reste, die das höhere Molekulargewicht als Isophoron bedingen, abspalten kann. Besonders bevorzugt stammt das erzeugte Isophoronnitril aus der Umsetzung von Isophoron und aus der Umsetzung mindestens eines keto- und ggf. hydroxylgruppenhaltigen Kohlenwasserstoffs mit einem Molekulargewicht von 178 - 276 g/mol mit Cyanwasserstoff.

In dem Wertstoffgemisch liegt neben Isophoron mindestens ein keto- und ggf. hydroxylgruppenhaltiger Kohlenwasserstoff mit einem Molekulargewicht von 178 - 276 g/mol vor. Bevorzugt liegen mindestens zwei, weiter bevorzugt mindestens drei, noch weiter bevorzugt mindestens vier, ganz besonders bevorzugt mindestens fünf, noch weiter bevorzugt mindestens sechs, noch weiter bevorzugt mindestens sieben, noch weiter bevorzugt mindestens acht und am meisten bevorzugt mindestens neun unterschiedliche keto- und ggf. hydroxylgruppenhaltige Kohlenwasserstoffe mit einem Molekulargewicht von 178 - 276 g/mol vor.

Bevorzugt weist mindestens einer der keto- und ggf. hydroxylgruppenhaltigen Kohlenwasserstoffe mit einem Molekulargewicht von 178 - 276 g/mol eine Struktur ausgewählt aus einer den nachfolgend abgebildeten Strukturformeln (I) bis (IX) auf:

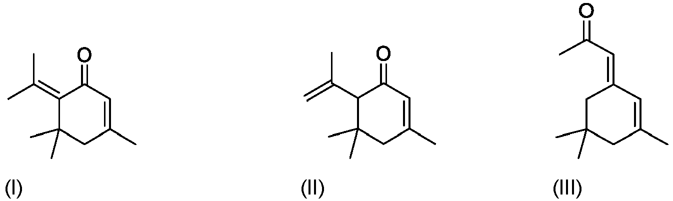

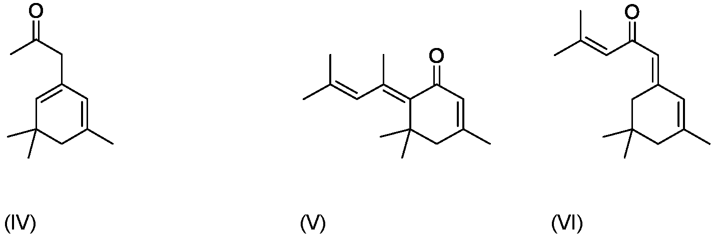

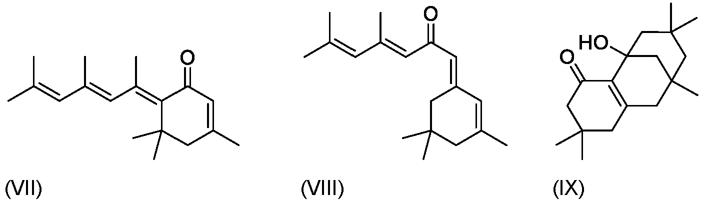

Bevorzugt weisen mindestens zwei, weiter bevorzugt mindestens drei, noch weiter bevorzugt mindestens vier, ganz besonders bevorzugt mindestens fünf, noch weiter bevorzugt mindestens sechs, noch weiter bevorzugt mindestens sieben, noch weiter bevorzugt mindestens acht und am meisten bevorzugt neun unterschiedliche keto- und ggf. hydroxylgruppenhaltige Kohlenwasserstoffe mit einem Molekulargewicht von 178 - 276 g/mol jeweils eine der Strukturen ausgewählt aus der Gruppe an Strukturformeln bestehend aus (I), (II), (III), (IV), (V), (VI), (VII), (VIII) und (IX) auf.

Bevorzugt beträgt der Massenanteil an keto- und ggf. hydroxylgruppenhaltigen Kohlenwasserstoffen mit einem Molekulargewicht von 178 - 276 g/mol an dem Wertstoffgemisch, bezogen auf die Gesamtmasse des Wertstoffgemischs vor Zugabe von Cyanwasserstoff, zwischen 1 und 50 Gew.-%, weiter bevorzugt zwischen 3 und 15 Gew.-%, noch weiter bevorzugt zwischen 4 und 10 Gew.-%.

Bevorzugt handelt es sich bei dem mindestens einen keto- und ggf. hydroxylgruppenhaltigen Kohlenwasserstoff mit einem Molekulargewicht von 178 - 276 g/mol um mindestens ein Überkondensat aus der Synthese von Isophoron aus der Aldolkondensation von Aceton. Entsprechende Überkondensate, die aus der Kondensation von vier oder mehr Acetonmolekülen bei der Aldolkondensation stammen, während für die Bildung von Isophoron nur drei Acetonmoleküle benötigt werden, wurden im Stand der Technik bislang nach Hydrolyse, Phasentrennung des Hydrolysats und Entfernen organischer Komponenten mit einer Molmasse von < 126 g/mol aus der organischen Phase der Phasentrennung über übliche Auftrennverfahren, insbesondere über Destillation, üblicherweise vom Zielprodukt Isophoron separiert. Ein bedeutsamer Aspekt der vorliegenden Erfindung ist somit, dass festgestellt wurde, dass es entgegen bisheriger Annahmen für die Synthese von Isophoronnitril aus Isophoron und Cyanwasserstoff von Vorteil ist, die anfallenden Überkondensate nicht zu entfernen, da sie die Isophoronnitril-Synthese überraschenderweise nicht negativ beeinflussen, sondern in ihrer Gegenwart sogar Ausbeuten und Selektivitäten steigen. Vorteilhaft ist weiterhin, dass der Energiebedarf über die Gesamtreaktion vermindert werden kann, wenn Abtrennung, insbesondere Destillation, der Isophoronreichen Fraktion unterbleiben kann.

Ein bevorzugter Gegenstand der vorliegenden Erfindung ist somit ein Verfahren zur Herstellung von Isophoronnitril, bei dem ein Gemisch umfassend die Wertstoffe Isophoron und mindestens einen keto- und ggf. hydroxylgruppenhaltigen Kohlenwasserstoff mit einem Molekulargewicht von 178 - 276 g/mol, das erhältlich ist über ein Verfahren, bei dem in der Reihenfolge der Schritte a) bis d)
a) basenkatalysiert Aceton zu Isophoron einer Aldolkondensation unterworfen,
b) der Wertstrom der Aldolkondensation hydrolysiert,
c) eine Phasentrennung des Hydrolysats durchgeführt, und
d) die aus der Phasentrennung stammende organische Phase von organischen Komponenten mit einer Molmasse von < 126 g/mol befreit wird,
mit Cyanwasserstoff zu Isophoronnitril umgesetzt wird. Bei dieser bevorzugten Ausführungsform ist das Gemisch umfassend die Wertstoffe Isophoron und mindestens einen keto- und ggf. hydroxylgruppenhaltigen Kohlenwasserstoff mit einem Molekulargewicht von 178 - 276 g/mol die aus der organischen Phasentrennung stammende organische Phase nach Abtrennung der organischen Komponenten mit einer Molmasse von < 126 g/mol. Insbesondere bei dieser erfindungsgemäßen Ausführungsform wird das aus der organischen Phase der Phasentrennung nach der Hydrolyse stammende Produktgemisch nach dem vergleichsweise wenig energieintensiven Entfernen organischer Komponenten mit einer Molmasse von < 126 g/mol direkt und ohne weitere Aufreinigung mit Cyanwasserstoff zu Isophoronnitril umgesetzt.

Die basenkatalysierte Aldolkondensationsreaktion a) von Aceton zu Isophoron wird bevorzugt in einer Flüssigphasenreaktion durchgeführt. Prinzipiell lässt sich Isophoron aber auch in der Gasphase oder in überkritischem Aceton aus Aceton herstellen.

Bevorzugt wird das Aceton in der flüssigen Phase bei Temperaturen von 100 - 250 °C, bevorzugt 150 - 250 °C, besonders bevorzugt 180 - 250 °C, und in einem Druckbereich von 5 - 50 bar, bevorzugt 10 - 50 bar, besonders bevorzugt von 20 - 50 bar zu Isophoron umgesetzt, wobei die angegebenen Werte beliebig miteinander kombiniert werden können.

In einer Gasphasenreaktion wird das Aceton innerhalb des verwendeten Reaktors durch katalytische Reaktion bevorzugt bei Temperaturen im Bereich von 100 bis 400 °C, bevorzugt 200 - 400 °C zu Isophoron umgesetzt.

Im überkritischen Bereich wird das Aceton innerhalb des verwendeten Reaktors durch katalytische Reaktion bevorzugt bei Temperaturen im Bereich von 250 bis 350 °C und einem Druckbereich von 50 bis 200 bar zu Isophoron umgesetzt.

Bei den als Basen eingesetzten Katalysatoren kann es sich um homogene oder heterogene Katalysatoren handeln. In der Flüssigphase wird bevorzugt ein homogener Katalysator eingesetzt. In der Gasphase wird bevorzugt ein heterogener Katalysator verwendet. Für die Reaktion im überkritischen Bereich können sowohl homogene als auch heterogene Katalysatoren verwendet werden.

Die Kondensation von Aceton zu Isophoron wird bevorzugt basisch katalysiert. Insbesondere in der flüssigen Phase ist die eingesetzte Base bevorzugt eine Alkalilauge. Noch weiter bevorzugt wird der basische Katalysator ausgewählt aus der Gruppe bestehend aus NaOH und KOH.

Bevorzugt beträgt der Gehalt an Base, insbesondere an Alkalilauge, weiter bevorzugt NaOH oder KOH, < 1 Gew.-%, bevorzugt < 0,5 Gew.-%, besonders bevorzugt < 0,2 Gew.-%, bezogen auf die Reaktionszusammensetzung. Ganz besonders bevorzugt wird als Katalysator NaOH in einer Menge von 0,015 bis 0,05 Gew.-% eingesetzt.

Die eingesetzte Wasserkonzentration ergibt sich u. a. aus den Rückführströmen der Aufarbeitungsprozesse. Bevorzugt beträgt der Wassergehalt des Reaktionsgemisches, bezogen auf die gesamte Flüssigkeitsmenge, kleiner oder gleich 30 Gew.-%, bevorzugt kleiner oder gleich 20 Gew.-%.

Der Acetongehalt im Reaktionsgemisch, bezogen auf die gesamte Flüssigkeitsmenge, beträgt bevorzugt größer oder gleich 60 Gew.-%, weiter bevorzugt größer oder gleich 70 Gew.-%, besonders bevorzugt größer oder gleich 75 Gew.-%.

Die Reaktion kann in beliebigen Reaktoren, bevorzugt in Rohrreaktoren, Rührkesseln, Rührkesselkaskaden, Festbettreaktoren, Druckdestillationsreaktoren/Reaktivdestillationsreaktoren, mikrostrukturierten Reaktoren, Schlaufenreaktoren oder in Kombinationen aus den genannten Reaktoren durchgeführt werden.

Der Begriff Druckdestillationsreaktor ist hier gleichzusetzen mit Apparaten, in denen eine Reaktivdestillation durchgeführt wird. Die Reaktivdestillation ist in der Fachliteratur hinlänglich beschrieben.

Nach Durchführung der Aldolkondensation wird der Wertstrom bevorzugt vom Reaktionsgemisch abgetrennt. Bevorzugt wird das Reaktionsgemisch aufgearbeitet und in die einzelnen Komponenten getrennt. Dabei handelt es sich neben Isophoron um Leichtsieder (insbesondere Aceton, Diacetonalkohol und Mesityloxid), höhere Kondensationsprodukte (Überkondensate) des Acetons (insbesondere Xylitone und Isoxylitone), Wasser und Katalysator.

Die Auftrennung in die Komponenten kann prinzipiell mit allen Trennmethoden, insbesondere Destillation, Entspannungsverdampfung, Kristallisation, Extraktion, Sorption, Permeation, Phasentrennung oder Kombinationen davon kontinuierlich oder absatzweise, ein- oder mehrstufig durchgeführt werden. Bevorzugt erfolgt die Trennung durch Destillation in einem oder mehreren Apparaten.

Dabei kann die Destillation räumlich getrennt von der Isophoronsynthese (Reaktion) durchgeführt werden oder in einem Apparat stattfinden. Bevorzugt erfolgt die Abtrennung der einzelnen Fraktionen durch eine Reaktivdestillation, bevorzugt in einer Reaktivdestillationskolonne.

Besonders bevorzugt wird die Abtrennung räumlich getrennt von der Isophoronsynthese (Reaktion) in einer Reaktivdestillationskolonne mit einer Seitenstromentnahme durchgeführt.

Bevorzugt wird die Abtrennung so durchgeführt, dass die folgenden drei Fraktionen erhalten werden:
i) Eine Fraktion aus nicht umgesetzten Aceton, Wasser und Leichtsiedern (insbesondere Diacetonalkohol und Mesityloxid)
ii) Eine Fraktion, in der insbesondere farbgebende Substanzen angereichert werden.
iii) Eine Fraktion umfassend insbesondere Isophoron, höherkondensierte Produkte, Wasser und Katalysator, genannt Wertstrom. Diese Fraktion wird anschließend einer Hydrolyse unterworfen.

Die Fraktion i) wird bevorzugt kondensiert und anschließend zur Reaktion in den Reaktor zurückgeführt. In einer weiter bevorzugten Ausführungsform wird sie als Brüdenstrom entnommen, enthaltend im Wesentlichen Aceton, Wasser und Leichtsieder, im Wesentlichen Diacetonalkohol und Mesityloxid, kondensiert und dem Reaktor mit den Einsatzstoffen Aceton, Wasser und gegebenenfalls Katalysator wieder zugesetzt.

Die Fraktion ii) wird in einer bevorzugten Ausführungsform als Seitenstrom der Destillationskolonne, bevorzugt einer Reaktivdestillationskolonne, entnommen, gegebenenfalls neutralisiert und weiter aufgearbeitet. Diese Fraktion wird weiter aufgereinigt und die enthaltenden Wertstoffe werden in den Prozess zurückgeführt.

Bei der Aufarbeitung können alle gängigen Trennmethoden wie z.B. Destillation, Entspannungsverdampfung, Kristallisation, Extraktion, Sorption, Permeation, Phasentrennung oder Kombinationen davon zum Einsatz kommen. Die Aufreinigung kann kontinuierlich oder absatzweise, ein- oder mehrstufig durchgeführt werden. Bevorzugt wird die Aufreinigung durch Destillation erreicht. Besonders bevorzugt wird die Aufreinigung durch eine Kombination aus Neutralisation oder Extraktion und anschließender Destillation, bevorzugt in einer Reaktivdestillationskolonne, erreicht. Bevorzugt wird die aufgearbeitete Phase mit den Wertprodukten aus Isophoron, Hochsiedern und gegebenenfalls Katalysator in die Hydrolyse geführt. Eine weitere erhaltene Phase aus Wertprodukten, im Wesentlichen enthaltend Aceton, Diacetonalkohol und Mesityloxid, wird bevorzugt in die Reaktion zurückgeführt. Gegebenenfalls anfallende Rückstände werden der thermischen Verwertung zugeführt.

Die Fraktion iii) fällt bevorzugt im Sumpf der Destillationskolonne an und kann von dort unter Erhalt des Wertstroms entnommen werden.

Der Wertstrom der Aldolkondensation wird nachfolgend bevorzugt b) hydrolysiert, d.h. einer Hydrolyse unterworfen. Das Ziel der Hydrolyse ist es, im Wertstrom enthaltene Nebenprodukte teilweise oder vollständig in Isophoron, Aceton, oder andere nutzbare Wertprodukte zu überführen. Die Hydrolyse kann in allen gängigen Reaktoren, Destillationskolonnen oder Kombinationen davon durchgeführt werden. Bevorzugt wird die Hydrolyse als Reaktivdestillation durchgeführt, in dem die entstehenden Leichtsieder, im Wesentlichen enthaltend Aceton, Diacetonalkohol und Mesityloxid, direkt aus der Hydrolysezone entfernt und in die Reaktion zurückgeführt werden, und somit nicht mehr für Nebenreaktionen in der Hydrolyse zur Verfügung stehen.

Ganz besonders bevorzugt kann die Hydrolyse der Fraktion iii) in einem Apparat, durch eine Reaktivdestillation durchgeführt, bevorzugt in einer Reaktivdestillationskolonne, wobei gleichzeitig die Trennung des Reaktionsgemisches in die Fraktionen i) bis iii) erfolgt, so dass die entstehenden Produkte gleichzeitig entsprechend aufgetrennt werden und die Fraktion iii) hydrolysiert wird.

Gegebenenfalls können die Hydrolyse und die destillative Abtrennung auch in einem Apparat mit der Isophoronsynthese (Reaktion) stattfinden.

Die Hydrolyse kann in allen Mischungsverhältnissen der organischen Komponenten mit Wasser mit oder ohne Katalysator durchgeführt werden. Die Wasserkonzentration in der Hydrolyse beträgt dabei 30,1 - 99,9 Gew.-% bezogen auf die Gesamtmasse der anwesenden Bestandteile. Im Falle der homogenen Katalyse wird bei der Hydrolyse bevorzugt derjenige Katalysator eingesetzt, der auch bei der Aldolkondensation verwendet wird. Bevorzugt werden Katalysatorkonzentrationen von 0,001 - 10 Gew.- %, besonders bevorzugt von 0,05 - 1 Gew.-%, bezogen auf die Gesamtmasse der Zusammensetzung, gewählt. Der Druck im Hydrolysereaktor beträgt 1-200 bar, bevorzugt 20 - 60 bar. Besonders bevorzugt wird die Hydrolyse mindestens bei dem Druck durchgeführt, der auch im Isophoronsyntheseschritt (Reaktion) herrscht. Die Temperatur der Hydrolyse beträgt 100 - 300 °C, bevorzugt 210 - 260 °C. Besonders bevorzugt wird sich bei Verwendung einer Reaktivdestillationskolonne eine Temperatur bzw. ein Temperaturverlauf einstellen, der den Siedetemperaturen im Sumpf und auf den einzelnen Trenn- oder Reaktionsstufen entspricht.

Die Hydrolyse kann in einem oder mehreren Apparaten einstufig oder mehrstufig durchgeführt werden.

Das erhaltene Hydrolysat wird anschließend bevorzugt, nachdem es weiter bevorzugt aus dem Hydrolysereaktor der Reaktivdestillationskolonne ausgeführt und abgekühlt wurde, einer Phasentrennung c) unterworfen.

Durch die Phasentrennung wird eine im Wesentlichen organische Fraktion i) und eine im Wesentlichen wässrige Fraktion ii) erhalten. Im Falle einer homogenen Katalyse der Aldolkondensation enthält die wässrige Fraktion ii) auch den Katalysator. Für die Phasentrennung können übliche Phasentrennbehälter mit und ohne Einbauten zum Einsatz kommen. Die Phasentrennung erfolgt bei einer Temperatur zwischen 0 - 200 °C, bevorzugt bei 0 - 100 °C, besonders bevorzugt bei 20 - 70 °C, und bei einem Druck von 1 - 150 bar, bevorzugt 20 - 60 bar, besonders bevorzugt bei dem Druck, der auch in der Hydrolyse herrscht.

Die im Wesentlichen organische Phase i) umfassend das Zielprodukt Isophoron wurde bislang ggf. neutralisiert und mit üblichen Methoden aufgereinigt, so dass ein Isophoron mit der gewünschten Reinheit und Farbstabilität erhalten wurde. Dabei konnten alle gängigen Trennmethoden, insbesondere Destillation, Entspannungsverdampfung, Kristallisation, Extraktion, Sorption, Permeation, Phasentrennung, sowie Kombinationen davon zum Einsatz kommen. Die Aufreinigung konnte kontinuierlich oder absatzweise, ein- oder mehrstufig, unter Druck oder im Vakuum durchgeführt werden. Bevorzugt wurde die Aufreinigung durch Destillation erreicht. Besonders bevorzugt wurde die Aufreinigung durch eine Kombination aus Neutralisation oder Extraktion und folgender Destillation erreicht.

Abweichend von der bisherigen Vorgehensweise wird nun die aus der Phasentrennung stammende organische Phase nur von organischen Komponenten mit einer Molmasse von < 126 g/mol befreit, bevor der verbleibende Rest der organischen Phase mit Cyanwasserstoff zu Isophoronnitril umgesetzt wird. Bevorzugt erfolgt die Abtrennung organischer Komponenten mit einer Molmasse von < 126 g/mol über ein einstufiges Verfahren.

Bevorzugt wird die organische Phase i) durch thermische Auftrennung von organischen Komponenten mit einer Molmasse von < 126 g/mol befreit. Weiter bevorzugt erfolgt dies durch Destillation oder Rektifikation bei einem Druck von 60 - 150 mbar, einer Sumpftemperatur von 130 - 170 °C und einer Kopftemperatur von 70 - 120 °C, wobei im Sumpf Isophoron, alle Komponenten mit einer größeren Molmasse als Isophoron (insbesondere die Kohlenwasserstoffe mit einem Molekulargewicht von 178 - 276 g/mol sowie Hochsieder mit einer Molmasse >276 g/mol) verbleiben. In einer ganz besonders bevorzugten Ausführungsform kann dieser Sumpf direkt zusammen mit zugesetztem Isophoron für das erfindungsgemäße Verfahren zur Herstellung von Isophoronnitril eingesetzt werden.

Die Fraktion enthaltend die Komponenten mit einer Molmasse von < 126 g/mol kann verworfen werden. In einer bevorzugten Ausführungsform wird sie jedoch nach Zusatz von Wasser einer erneuten Phasentrennung unterzogen. Anschließend wird die dabei erhaltene wässrige Phase der Aldolkonsation, der Hydrolyse oder der Abwasserbehandlung (siehe nachfolgender Absatz) und die erhaltene organische Fraktion einer thermischen Verwertung zugeführt.

Die im Wesentlichen wässrige Fraktion ii) kann einer Abwasseraufreinigung zugeführt werden. Hier erfolgt die Trennung des Reaktionswassers als Hauptbestandteil und gegebenenfalls des Katalysators von gegebenenfalls noch gelösten organischen Komponenten, wie z. B. Isophoron, Aceton und höher kondensierten Produkten. Die Abwasseraufreinigung wird bevorzugt in einer oder mehreren Destillationskolonnen durchgeführt.

Erfindungsgemäß wird das Gemisch umfassend die Wertstoffe Isophoron und mindestens einen keto- und ggf. hydroxylgruppenhaltiger Kohlenwasserstoff mit einem Molekulargewicht von 178 - 276 g/mol mit Cyanwasserstoff zu Isophoronnitril umgesetzt. Bevorzugt wird die Umsetzung des wertstoffhaltigen Gemisches mit Cyanwasserstoff basenkatalysiert durchgeführt, da die basischen Katalysatoren freie Cyanid-Ionen für die 1,4-Additionsreaktion zur Verfügung stellen.

Bevorzugte Katalysatoren können ausgewählt werden aus der Gruppe bestehend aus basischen Alkali- oder Erdalkaliverbindungen, Oniumverbindungen, organischen Basen und Phasentransfer-katalysatoren. Ganz besonders bevorzugt kann der Katalysator ausgewählt werden aus der Gruppe bestehend aus basischen Alkali- oder Erdalkalimetallverbindungen. Noch weiter bevorzugt kann der Katalysator ausgewählt werden aus der Gruppe bestehend aus Alkalialkoholaten, -oxiden, -hydroxiden, -carbonaten, -peroxiden und -cyaniden, sowie Erdalkalialkoholaten, -oxiden, -hydroxiden, -peroxiden und -cyaniden.

Die Umsetzung mit Cyanwasserstoff (HCN) kann in Lösung oder lösemittelfrei durchgeführt werden.

Die Katalyse kann homogen oder heterogen katalysiert werden. Bei der homogenen Katalyse liegen Katalysator und Reaktanden in der gleichen Phase vor. Bei der heterogenen Katalyse liegen Katalysator und Reaktanden in unterschiedlichen Phasen vor.

Bevorzugt wird bei dem erfindungsgemäßen Verfahren eine homogene Basenkatalyse eingesetzt. Hierzu werden ganz besonders bevorzugt Alkalialkoholate, insbesondere Natriummethanolat, als Katalysatoren eingesetzt.

Der Cyanwasserstoff kann in Reinform, aber auch als Gemisch mit Stabilisatoren eingesetzt werden. Als Stabilisatoren eignen sich alle dem Fachmann für diesen Zweck bekannten Verbindungen, beispielsweise Phosphorsäure, Schwefeldioxid und Oxalsäure. Bevorzugt werden Phosphorsäure, Schwefeldioxid oder Gemische hieraus als Stabilisatoren für die Blausäure eingesetzt. Der Anteil der Stabilisatoren liegt üblicherweise im Bereich von 0,1 bis 2 Gew.-%, bezogen auf die eingesetzte Blausäure, vorzugsweise im Bereich von 0,5 bis 1 Gew.-%.

Die Umsetzung kann in Gegenwart oder Abwesenheit von inerten Lösungsmitteln vorgenommen werden. Besonders bevorzugt verwendet man Isophoron im molaren Überschuss, bezogen auf die eingesetzte Blausäure und setzt kein externes Lösungsmittel zu.

Generell wird das erfindungsgemäße Verfahren derart durchgeführt, dass ein Überschuss an Isophoron eingesetzt wird, da so eine höhere Selektivität zu Isophoronnitril erzielt wird. Bevorzugt beträgt das molare Verhältnis Isophoron/HCN > 1 : 1, weiter bevorzugt 19 : 1 bis 1,5 : 1, vorzugsweise 3 : 1 bis 1,5 : 1.

Die Katalysatorkonzentration, bezogen auf die eingesetzte Isophoronmenge, liegt im Bereich von 0,03 bis 20 Gew.-%, vorzugsweise im Bereich von 0,03 bis 1 Gew.-% .

Bevorzugt wird die Gesamtmenge des Isophorons vorgelegt und auf die gewünschte Reaktionstemperatur gebracht, bevor der Cyanwasserstoff in Anwesenheit des Katalysators zugegeben wird.

Die HCN-Zudosierung erfolgt vorzugsweise so, dass eine homogene Verteilung der Edukte (Isophoron und HCN) gewährleistet wird. Die HCN-Zudosierung kann auf übliche, dem Fachmann bekannte Weise erfolgen, bevorzugt über Statikmischer, Pumpen oder Verdüsung.

Die Reaktionstemperaturen liegen üblicherweise im Bereich von 130 bis 225 °C, bevorzugt im Bereich von 135 bis 195 °C und ganz besonders bevorzugt im Bereich von 140 bis 175 °C.

Die Reaktion wird üblicherweise bei Drücken von 0,01 bis 10 bar, bevorzugt 1 bis 5 bar, besonders bevorzugt bei Normaldruck (Atmosphärendruck) durchgeführt.

Innerhalb der vorstehend dargelegten Reaktionsparameter wird das HCN bevorzugt so zudosiert, dass eine ausreichend niedrige Konzentration von HCN resultiert und somit eine hohe Selektivität sowie ein hoher Umsatz zu Isophoronnitril erzielt werden kann.

Vorzugsweise werden die Konzentrationen an nicht umgesetztem, freiem HCN und die Gesamtkonzentration an Cyanidionen (Summe aus freiem HCN und als Cyanhydrine von Isophoron und Isophoronnitril gebundenem Cyanid) bestimmt und die Reaktionsbedingungen so angepasst, dass eine optimierte Selektivität hinsichtlich IPN erhalten wird. Die Bestimmung der genannten Cyanidionen-Konzentrationen erfolgt vorzugsweise durch Titration.

Das erfindungsgemäße Verfahren kann in bevorzugter Weise kontinuierlich, diskontinuierlich oder semikontinuierlich durchgeführt werden, insbesondere kontinuierlich.

In bevorzugter Ausführung wird die Reaktionsdurchführung, je nach eingesetztem Katalysator, in einem Rührkessel, einer Rührkesselkaskade, einem Kreislaufreaktor, einem Strömungsrohr, einem oder mehreren Festbettreaktoren oder einer Kolonne durchgeführt.

Vorzugsweise wird überschüssiges Isophoron durch Destillation abgetrennt und vorteilhafterweise erneut eingesetzt. Dazu wird das abgetrennte Isophoron mit frischem Isophoron vermischt und in den Reaktionsreaktor zurückgeführt. Das gebildete Isophoronnitril wird durch destillative Aufarbeitung von möglichen Nebenkomponenten abgetrennt und kann weiter eingesetzt werden.

Bevorzugt kann das mit dem Verfahren erhältliche Isophoronnitril eingesetzt werden für die Synthese von Isophorondiamin über aminierende Hydrierung. Gegenstand der vorliegenden Erfindung ist somit auch die Verwendung des nach dem erfindungsmäßen Verfahrens erhältlichen Isophoronnitrils für die Synthese von Isophorondiamin.

Alle angegebenen Prozent (%) sind, wenn nicht anders angegeben, Massenprozent.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Beispiel 1:

### 1. Herstellung von Isophoronnitril ausgehend von Roh-Isophoron und HCN im Batchverfahren

Die Umsetzung von Roh-Isophoron zu Isophoronnitril wurde in einem 1 L Reaktor mit Rührer und Tropftrichter durchgeführt. 464 g Roh-Isophoron (aus Hydrolyse nach Phasentrennung und Abtrennung von Material mit Molmasse < 126 g/mol, 89,5 Gew.-% Isophoron, 8,8 Gew.-% Massenbereich 178 - 276 g/mol, 1,6 Gew.-% unidentifizierte Komponenten, siehe Tabelle 1) wurde in dem Reaktor vorgelegt und auf 20 °C temperiert. Eine Mischung aus 18,8 g Roh-Isophoron (aus Hydrolyse nach Phasentrennung und Abtrennung von Material mit Molmasse < 126 g/mol, 89,5 Gew.-% Isophoron, 8,8 Gew.-% Massenbereich 178 - 276 g/mol, 1,6 Gew.-% unidentifizierte Komponenten, siehe folgende Tabelle) und 40 g HCN wurden bei -6 °C in den Tropftrichter gefüllt. Der Reaktor wurde auf 175 °C erwärmt, der Katalysator Natriummethoxid (30 Gew.-% Lösung in Methanol) wurde zu dem im Reaktor vorgelegten Isophoron gegeben und die Katalysatorvorlage mit 5 g Roh-Isophoron (aus Hydrolyse nach Phasentrennung und Abtrennung von Material mit Molmasse < 126 g/mol, 89,5 Gew.-% Isophoron, 8,8 Gew.-% Massenbereich 178 - 276 g/mol, 1,6 Gew.-% unidentifizierte Komponenten, siehe Tabelle 1) gespült. Anschließend wurde die Zugabe der HCN/Roh-Isophoronmischung gestartet. Die Zugabe erfolgt über 30 Minuten bei 175 °C und Normdruck. Nach vollständiger Zugabe wurde die Reaktionsmischung für weitere 30 Minuten am Rückfluss bei 175 °C nachgerührt. Es wurde ein Isophoronumsatz von 37,6% (berechnet bezogen auf die Ausgangskonzentration Isophoron im Roh-Isophoron) und eine Selektivität für Isophoronnitril von 92,8% beobachtet (bezogen auf die Ausgangskonzentration Isophoron im Roh-Isophoron).

### 2. Herstellung von Isophoronnitril ausgehend von einer Mischung aus reinem Isophoron und Roh-Isophoron (1:1) und HCN im Batchverfahren

Die Umsetzung von Isophoron zu Isophoronnitril wurde in einem 1 L Reaktor mit Rührer und Tropftrichter durchgeführt. 464 g Isophoron (Mischung aus Rein- und Roh-Isophoron 1:1, 94,6 Gew.-% Isophoron, 4,5 Gew.-% Massenbereich 178 - 276 g/mol, 0,9 Gew.-% unidentifizierte Komponenten, siehe Tabelle 1; das Roh-Isophoron ist dasselbe wie in Beispiel 1) wurde in dem Reaktor vorgelegt und auf 20 °C temperiert. Eine Mischung aus 18,8 g Isophoron (Mischung aus Rein- und Roh-Isophoron 1:1, 94,6 Gew.-% Isophoron, 4,5 Gew.-% Massenbereich 178 - 276 g/mol, 0,9 Gew.-% unidentifizierte Komponenten, siehe Tabelle 1; das Roh-Isophoron ist dasselbe wie in Beispiel 1) und 40 g HCN wurden bei -6 °C in den Tropftrichter gefüllt. Der Reaktor wurde auf 175 °C erwärmt, der Katalysator Natriummethoxid (30 Gew.-% Lösung in Methanol) wurde zu dem im Reaktor vorgelegten Isophoron gegeben und die Katalysatorvorlage mit 5 g Isophoron (Mischung aus Rein- und Roh-Isophoron 1:1, 94,6 Gew.-% Isophoron, 4,5 Gew.-% Massenbereich 178 - 276 g/mol, 0,9 Gew.-% unidentifizierte Komponenten, siehe Tabelle 1; das Roh-Isophoron ist dasselbe wie in Beispiel 1) gespült. Anschließend wurde die Zugabe der HCN/Isophoronmischung gestartet. Die Zugabe erfolgt über 30 Minuten bei 175 °C und Normdruck. Nach vollständiger Zugabe wurde die Reaktionsmischung für weitere 30 Minuten am Rückfluss bei 175 °C nachgerührt. Es wurde ein Isophoronumsatz von 38,7% (berechnet bezogen auf die Ausgangskonzentration Isophoron im Roh-Isophoron) und eine Selektivität für Isophoronnitril von 93,5% beobachtet (bezogen auf die Ausgangskonzentration Isophoron im Roh-Isophoron).

### 3. Herstellung von Isophoronnitril ausgehend von reinem Isophoron und HCN im Batchverfahren

Die Umsetzung von Isophoron zu Isophoronnitril wurde in einem 1 L Reaktor mit Rührer und Tropftrichter durchgeführt. 464 g Isophoron (nach Feindestillation, 99,7 Gew.-% Isophoron, 0,1 Gew.-% Massenbereich 178 - 276 g/mol, 0,2 Gew.-% unidentifizierte Komponenten, siehe Tabelle 1) wurde in dem Reaktor vorgelegt und auf 20 °C temperiert. Eine Mischung aus 18,8 g Isophoron (nach Feindestillation, 99,7 Gew.-% Isophoron, 0,1 Gew.-% Massenbereich 178 - 276 g/mol, 0,2 Gew.-% unidentifizierte Komponenten, siehe Tabelle 1) und 40 g HCN wurden bei -6 °C in den Tropftrichter gefüllt. Der Reaktor wurde auf 175 °C erwärmt, der Katalysator Natriummethoxid (30 Gew.-% Lösung in Methanol) wurde zu dem im Reaktor vorgelegten Isophoron gegeben und die Katalysatorvorlage mit 5 g Isophoron (nach Feindestillation, 99,7 Gew.-% Isophoron, 0,1 Gew.-% Massenbereich 178 - 276 g/mol, 0,2 Gew.-% unidentifizierte Komponenten, siehe Tabelle 1) gespült. Anschließend wurde die Zugabe der HCN/Isophoronmischung gestartet. Die Zugabe erfolgt über 30 Minuten bei 175 °C und Normdruck. Nach vollständiger Zugabe wurde die Reaktionsmischung für weitere 30 Minuten am Rückfluss bei 175 °C nachgerührt. Es wurde ein Isophoronumsatz von 40,7% (berechnet bezogen auf die Ausgangskonzentration Isophoron im Roh-Isophoron) und eine Selektivität für Isophoronnitril von 78,3% beobachtet (bezogen auf die Ausgangskonzentration Isophoron im Roh-Isophoron).

**Tabelle 1**

| Material | Exp. 1 (Roh-IP) | Exp. 2 (1:1 Gemisch Rein- und Roh-IP) | Exp. 3 (Rein-IP) |
|---|---|---|---|
| Eduktzusammensetzung | | | |
| Isophoron | 89,5 | 94,6 | 99,7 |
| Massenbereich 178 - 276 g/mol | 8,8 | 4,5 | 0,1 |
| Unidentifizierte Komponente | 1,6 | 0,9 | 0,2 |
| | | | |

| Produktzusammensetzung | Massenanteil [%] auf Basis GC | | |
|---|---|---|---|
| Isophoron | 51,9 | 55,9 | 59,0 |
| Isophoronnitril | 34,9 | 36,2 | 31,9 |
| Massenbereich 178 - 276 g/mol | 7,5 | 5,6 | 6,8 |
| Unidentifizierte Komponente | 5,7 | 2,3 | 2,4 |
| Umsatz | 37,6 | 38,7 | 40,7 |
| Selektivität | 92,8 | 93,5 | 78,3 |

## Patentansprüche

1. Verfahren zur Herstellung von Isophoronnitril, bei dem ein Gemisch umfassend die Wertstoffe
a) Isophoron und
b) mindestens einen keto- und ggf. hydroxylgruppenhaltigen Kohlenwasserstoff mit einem Molekulargewicht von 178 - 276 g/mol
mit Cyanwasserstoff zu Isophoronnitril umgesetzt wird.

2. Verfahren nach Anspruch 1,
**dadurch gekennzeichnet, dass**
das Gemisch mindestens zwei unterschiedliche keto- und ggf. hydroxylgruppenhaltige Kohlenwasserstoffe mit einem Molekulargewicht von 178 - 276 g/mol umfasst.

3. Verfahren nach Anspruch 1 oder 2,
**dadurch gekennzeichnet, dass**
mindestens einer der keto- und ggf. hydroxylgruppenhaltigen Kohlenwasserstoffe mit einem Molekulargewicht von 178 - 276 g/mol eine Struktur ausgewählt aus einer den nachfolgend abgebildeten Strukturformeln (I) bis (IX) aufweist:

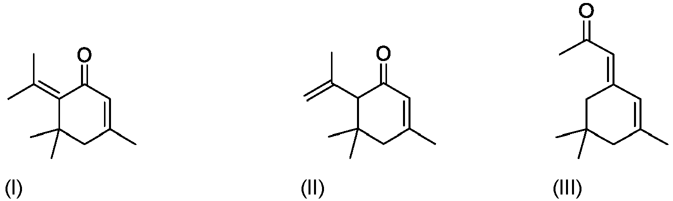

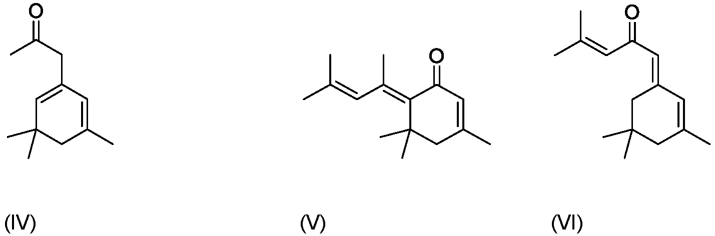

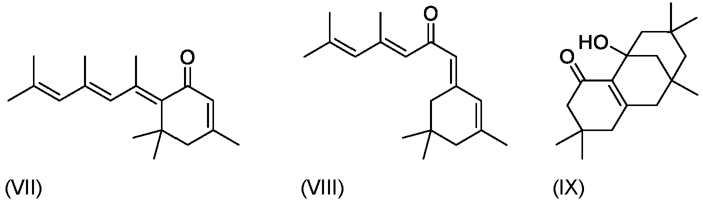

4. Verfahren nach einem der vorhergehenden Ansprüche,
**dadurch gekennzeichnet, dass**
der Massenanteil an keto- und ggf. hydroxylgruppenhaltigen Kohlenwasserstoffen mit einem Molekulargewicht von 178 - 276 g/mol an dem Wertstoffgemisch, bezogen auf die Gesamtmasse von Isophoron und keto- und ggf. hydroxylgruppenhaltigen Kohlenwasserstoffen mit einem Molekulargewicht von 178 - 276 g/mol, zwischen 1 und 50 Gew.-% beträgt.

5. Verfahren nach einem der vorhergehenden Ansprüche,
**dadurch gekennzeichnet, dass**
das Gemisch umfassend die Wertstoffe Isophoron und mindestens einen keto- und ggf. hydroxylgruppenhaltigen Kohlenwasserstoff mit einem Molekulargewicht von 178 - 276 g/mol erhältlich ist über ein Verfahren, bei dem in der Reihenfolge der Schritte a) bis d)
a) basenkatalysiert Aceton zu Isophoron einer Aldolkondensation unterworfen,
b) der Wertstrom der Aldolkondensation hydrolysiert,
c) eine Phasentrennung des Hydrolysats durchgeführt, und
d) die aus der Phasentrennung stammende organische Phase von organischen Komponenten mit einer Molmasse von < 126 g/mol befreit wird.

6. Verfahren nach Anspruch 5,
**dadurch gekennzeichnet, dass**
die Aldolkondensation a) bei einer Temperatur von 100 - 250 °C und einem Druck von 5 - 50 bar in der flüssigen Phase durchgeführt wird.

7. Verfahren nach Anspruch 5 oder 6,
**dadurch gekennzeichnet, dass**
die Hydrolyse des Wertstroms b) bei einer Temperatur von 210 - 260 °C und einem Druck von 20 - 60 bar durchgeführt wird.

8. Verfahren nach einem der Ansprüche 5 bis 7,
**dadurch gekennzeichnet, dass**
die Phasentrennung bei einer Temperatur von 0 - 100 °C und einem Druck von 20 - 60 bar erfolgt.

9. Verfahren nach einem der Ansprüche 5 bis 8,
**dadurch gekennzeichnet, dass**
die organische Phase i) durch thermische Auftrennung von organischen Komponenten mit einer Molmasse von < 126 g/mol befreit wird.

10. Verfahren nach einem der vorhergehenden Ansprüche,
**dadurch gekennzeichnet, dass**
die Umsetzung mit Cyanwasserstoff basenkatalysiert durchgeführt wird.

11. Verfahren nach Anspruch 10,
**dadurch gekennzeichnet, dass**
die Umsetzung mit Cyanwasserstoff homogen katalysiert mit mindestens einem Alkalialkoholat als Katalysator erfolgt.
